Europäisches Patentamt

European Patent Office

Office européen des brevets

(19)

(11) Veröffentlichungsnummer: **0 386 753**

**A2**

(12) # EUROPÄISCHE PATENTANMELDUNG

(21) Anmeldenummer: **90104421.4**

(22) Anmeldetag: **08.03.90**

(51) Int. Cl.⁵: **A61M 5/14, B65D 23/10**

(30) Priorität: **10.03.89 DE 3907862**

(43) Veröffentlichungstag der Anmeldung:
**12.09.90 Patentblatt 90/37**

(84) Benannte Vertragsstaaten:
**AT BE CH FR GB IT LI NL SE**

(71) Anmelder: **Herbert, Reinhold**
**Breite Strasse 7**
**D-6392 Neu-Anspach 1(DE)**

(72) Erfinder: **Herbert, Reinhold, Dipl.-Chem.-Ing.**
**Breite Strasse 7**
**D-6392 Neu-Anspach 1(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**D-6200 Wiesbaden(DE)**

(54) **Über Kopf aufzuhängender Behälter mit einem Etikett.**

(57) Die Erfindung betrifft einen über Kopf aufzuhängenden Behälter (1) aus Glas oder Kunststoff, insbesondere.eine Flasche zum Verabreichen von medizinischen Flüssigkeiten an einen Patienten, mit einem Etikett (2), auf dessen Rückseite eine stark haftende Klebeschicht aufgebracht ist. Dabei reicht das Etikett (2) mit seiner dem Behälterboden (3) zugewandten Seite bis an die Bodenebene (4) heran und umgibt mit seiner Breite mindestens die Hälfte des Behälterumfanges. Das Etikett (2) ist parallel zur Bodenebene (4) nahezu über seine gesamte Breite geschlitzt, so daß ein Etikettstreifen (6) gebildet wird. Der sich vom Schlitz (5) senkrecht parallel zur Bodenebene (4) erstreckende Etikettstreifen (6) ist über den Behälterboden (3) wegklappbar ausgebildet und an einer Aufhängevorrichtung (8) aufhängbar.

EP 0 386 753 A2

## Über Kopf aufzuhängender Behälter mit einem Etikett

Die Erfindung betrifft einen über Kopf aufzuhängenden Behälter aus Glas oder Kunststoff, insbesondere Flasche zum Verabreichen von medizinischen Flüssigkeiten an einen Patienten, mit einem Etikett, auf dessen Rückseite eine stark haftende Klebeschicht aufgebracht ist.

Die Notwendigkeit, Flaschen über Kopf aufzuhängen, besteht in vielen Bereichen des täglichen Lebens, nicht nur im industriellen und medizinischen Bereich, sondern vielfach auch im privaten Bereich. Insbesondere finden jedoch über Kopf aufgehängte Flaschen Verwendung im medizinischen, klinischen Bereich, z.B. bei Infusionen, Sonderernährung (stationär) usw.. Dabei ist es wesentlich, daß die Flaschen sicher und einfach aufgehängt und in diesem Zustand noch leicht und einwandfrei gehandhabt werden können. Solche Flaschen sind in der Regel mit einem aufgeklebten Etikett versehen, auf denen Informationen über den Flascheninhalt, Hersteller, Indikationsweise usw. aufgedruckt sind.

Vorrichtungen, Flaschen über Kopf aufzuhängen, sind an sich bekannt. So werden z.B. hohlzylindrisch ausgebildete Netze, die am vorderen Ende einen Ring und am hinteren Ende eine Zugschnur und eine Aufhängeschnur aufweisen, verwendet. Diese Art der Vorrichtung gestattet jedoch aus Stabilitätsgründen jeweils lediglich die Verwendung einer bestimmten Flaschengröße. Darüberhinaus erschweren sie durch die netzartige Umhüllung der Flasche ein genaues und schnelles Ablesen der in der Flasche verbleibenden Menge an Flüssigkeit oder Feststoff. Im Hinblick auf diese Nachteile und in Anbetracht des hohen Preises dieser Netze sind diese Netze nicht besonders zweckdienlich.

Eine weitere bekannte Vorrichtung zum über Kopf aufhängen von Flaschen besteht in einem Kunststoffbeutel, der an einem Ende zum Einschieben der aufzuhängenden Flasche offen ist und Aufhängeösen aufweist. Am anderen Ende besitzt dieser Beutel eine kreisförmige Öffnung zur Aufnahme des Flaschenhalses.

Das DE-GM 79 17 315 beschreibt eine spiralförmige Aufhängevorrichtung. Diese Vorrichtung umfaßt einen Haltering für den Flaschenhals und einen Haltering für den Flaschenbauch, die im Lagerzustand in einer Ebene angeordnet sind, wobei der Haltering für den Flaschenhals von dem Haltering für den Flaschenbauch umgeben ist, die beiden Ringe über zwei Stege verbunden sind und der Haltering für den Flaschenbauch eine ebenfalls im Lagerzustand in der Ebene liegende Aufhängeeinrichtung aufweist, die im Lagerzustand ihrerseits den Haltering für den Flaschenbauch umgibt und mit diesem mittels Stegen verbunden ist. Im Gebrauchszustand wird die Flasche mit dem Hals durch den Haltering für den Flaschenhals gesteckt und die Vorrichtung so über die Flasche gezogen, daß der Haltering für den Flaschenbauch den Flaschenbauch umgibt und die Aufhängeeinrichtung zum Aufhängen frei bleibt. Die Vorrichtung umgibt im Gebrauchszustand die Flasche spiralförmig.

Eine weitere Vorrichtung ist in dem DE-GM 74 42 831 beschrieben und weist jeweils einen Haltering für den Hals einer Flasche und einen Haltering für den Bauch einer Flasche auf, wobei zusätzlich noch zwei Aufhängeösen vorgesehen sind, die am Ring für den Flaschenbauch angreifen.

Ähnliche Flaschenaufhängevorrichtungen sind in der DE-OS 21 28 993, DE-OS 25 48 726 und DE-OS 33 10 841 beschrieben, die als einstückige Kunststofformteile mit einem dem Flaschenhals und einem dem Flaschenumfang angepaßten Halteteil ausgebildet sind, wobei sich an dem dem Flaschenumfang angepaßten Halteteil ein dieses umgebendes Aufhängeteil anschließt.

Allen diesen Lösungen sind die Nachteile gemeinsam, daß sie material- und fertigungstechnisch aufwendig herzustellen sind und zur Aufhängung selbst eine Aufhängevorrichtung als separates Teil benötigen, die in der Regel schlecht handhabbar ist.

Die DE-OS 36 31 021 zeigt ein Gehänge für einen Behälter, auf welchen ein Selbstklebeetikett aufgebracht ist, das an seiner dem Boden des Behälters zugewandten Seite mit einer Aufhängelasche versehen ist. Die Aufhängelasche steht somit beim Aufbringen des Etikettes über den Behälterboden des Behälters über und muß nachfolgend umgelegt und gegen den Behälterboden fixiert werden, um zu verhindern, daß die Lasche während des Transportes des Behälters beschädigt wird. Dabei erweist es sich als nachteilig, daß das Umlegen und Befestigen der Lasche einen erheblichen apparatetechnischen Aufwand erfordert, so daß dieses Gehänge nicht mit üblichen Etikettiermaschinen auf Behälter aufbringbar ist. Weiterhin besteht die Gefahr, daß die Aufhängelasche während des Transportes oder der Lagerung beschädigt wird. Ein weiterer Nachteil besteht darin, daß das Etikett selbst sehr kompliziert ausgebildet ist und eine hohe Menge Material verbraucht.

Die DE-OS 21 35 046 zeigt eine Dose mit einem Griff, welcher dadurch gebildet wird, daß ein Teil einer mechanisch an dem Behälter fixierten, ringförmigen Hülse hochgeklappt wird. Der restliche Bereich der Hülse dient somit als Lagerung, während der Griff scharnierartig umschwenkbar ist. Es handelt sich hiermit um ein gattungsgemäß unterschiedliches Aufhängesystem mit einem gelenkig gelagerten, laschenförmigen Griff. Der Nachteil besteht insbesondere darin, daß die mechanische Fixierung der Hülse einen hohen Aufwand erfordert. Weiterhin stellt die Hülse ein separates, zusätzliches Bauelement dar, welches zusätzlich gelagert und befestigt werden muß.

Es ist daher Aufgabe der Erfindung, einen über Kopf aufzuhängenden Behälter mit einem Etikett zu schaffen, wobei das Etikett als Aufhängevorrichtung dient und dabei billig und fertigungstechnisch leicht herstellbar und handhabbar ist.

Erfindungsgemäß wird die Aufgabe dadurch gelöst, daß das Etikett mit seiner dem Behälterboden zugewandten Seite bis an die Bodenebene heranreicht und mit seiner Breite mindestens die Hälfte des Behälterumfanges umgibt, und das parallel zur Bodenebene nahezu über seine gesamte Breite geschlitzt ist, so daß ein Etikettstreifen gebildet wird, wobei sich der vom Schlitz senkrecht parallel zur Bodenebene erstreckende Etikettstreifen über den Behälterboden wegklappbar ausgebildet und an einer Aufhängevorrichtung aufhängbar ist und daß der Etikettstreifen ohne Klebeschicht ausgebildet ist.

Damit der über Kopf aufzuhängende Behälter in besonderen Einsatzfällen eine bestimmte Schräglage einnehmen kann, umgibt das Etikett mit seiner Breite bis zu zwei Drittel des Behälterumfanges. In vorteilhafter Weise ist der abklappbare Etikettstreifen ohne Klebeschicht ausgebildet.

Durch die erfindungsgemäße Lösung kann das auf dem über Kopf aufzuhängenden Behälter angebrachte Etikett zum Aufhängen des Behälters verwendet werden, in dem der sich zur Bodenebene erstreckende Etikettstreifen, der nicht wie das übrige Etikett mit dem Flaschenkörper verbunden ist, derart abgeklappt werden kann, daß der Etikettstreifen den Behälterboden überspannt und dabei einen Abstand zwischen sich und dem Behälterboden bildet, in den die Aufhängevorrichtung eingreifen kann.

In einer besonderen Ausbildungsweise kann der sich vom Schlitz senkrecht parallel zur Bodenebene erstreckende Etikettstreifen mit einer Verstärkungsschicht, vorzugsweise aus einer textilen Schicht bestehend, ausgebildet sein, die vorzugsweise mit den Etikettstreifen verklebt ist.

Zur Vermeidung von Einrissen am Etikett ist am jeweiligen Schlitzende eine Ausnehmung vorgesehen.

Vorzugsweise besteht das Etikett aus Polyethylen, Polypropylen oder Polyurethan bzw. aus Laminaten mit Polyester oder Polyamid oder aus kunststoffkaschiertem Papier. Die Tragfähigkeit des Etikettstreifens wird dabei durch das Behältervolumen gemäß DIN 58369 mit 30 bzw. 50 N bestimmt. In Abhängigkeit von der Foliendicke und vom verwendeten Folienmaterial für das Etikett weist dieses eine Reißfestigkeit von etwa 40 bis etwa 110 N/mm² und eine Reißkraft von etwa 30 bis etwa 120 N/15 mm auf.

Durch das preisgünstige und leicht herstellbare Etikett ist es möglich, für jede Flaschengröße eine entsprechende Etikettgröße zu wählen.

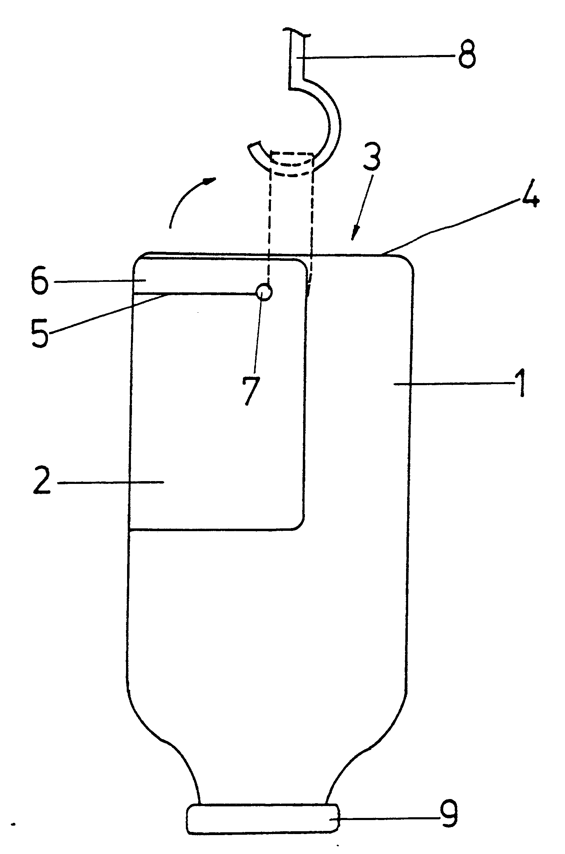

An einem Ausführungsbeispiel soll die Erfindung unter Darlegung weiterer Einzelheiten und der entsprechenden Vorteile näher erläutert werden. In der beigefügten Zeichnung ist ein Behälter in über Kopf aufgehängter Form dargestellt, wobei die strichlierten Linien den in Pfeilrichtung abgeklappten Etikettstreifen kennzeichnen, der an einer Aufhängevorrichtung aufgehängt ist.

An der Außenfläche des Behälters 1 ist ein Etikett 2 aufgebracht. Auf diesem Etikett 2 sind Informationen über den Inhalt des Behälters, den Hersteller, die Indikationsweise der im Behälter befindlichen Flüssigkeit usw. aufgedruckt. Dieses Etikett 2 besteht aus Polyethylen, Polypropylen oder Polyurethan bzw. aus Laminaten mit Polyester oder Polyamid oder aus kunststoffkaschiertem Papier. Die Dicke der Etikettfolie ist dabei so gewählt, daß das Etikett 2 eine Reißfestigkeit von etwa 40 bis etwa 110 N/mm und eine Reißkraft von etwa 30 bis 120 N/15 mm aufweist. Das Folienmaterial des Etiketts 2 muß dabei zäh, dehnbar, siegelfähig, bedruckbar, kältefest, chemikalienbeständig, physiologisch unbedenklich, äußerst rückschrumpfarm und formstabil sein. Darüberhinaus muß es eine gute Ein- und Weiterreißfestigkeit sowie eine gute Durchstoßfestigkeit aufweisen.

Das Etikett 2 reicht mit seiner dem Behälterboden 3 zugewandten Seite bis an die Bodenebene 4. Mit seiner Breite umgibt das Etikett 2 etwa die Hälfte bis zwei Drittel des Umfanges des Behälters 1. Umsomehr das Etikett 2 die Oberfläche des Behälters 1 bis an die Zweidrittelgrenze umgibt, umsomehr kann in besonderen Anwendungsfällen durch die Schwerkraft des Behälters 1 eine Schräglage des Behälters erreicht werden. Dies kann beispielsweise dann der Fall sein, wenn der Behälter 1 eine Form aufweist, bei der gewährleistet sein muß, daß die im Behälter befindliche Flüssigkeit vollständig über den sich verengenden Flaschenhals ausströmen kann.

Es versteht sich, daß für unterschiedliche Behälterdurchmesser bzw. Behältergrößen unterschiedlich dimensionierte Etiketten 2 verwendet werden.

Das Etikett 2 ist mit einem Schlitz 5 versehen, der parallel zur Bodenebene führt und nahezu über die gesamte Etikettbreite verläuft. Dadurch bildet sich ein Etikettstreifen 6, wobei das Etikett 2 vom Schlitz 5 bis zum von der dem Behälterboden 3 abgewandten Kante des Etikettes 2 gebildete Bereich auf seiner Rückseite mit einer Klebeschicht versehen ist, die mit der Außenoberfläche des Behälters 1, wie oben beschrieben, in Verbindung gebracht wird. Die Rückseite des Etikettstreifens 6 ist dabei ohne Klebeschicht. Die Aufbringung der Klebeschicht erfolgt dabei bereits herstellerseitig, wobei die Klebeschicht mit einer silikonisierten Papierfolie abgedeckt sein kann und das Etikett 2 in dieser Form an den Behälterhersteller bzw. Behälterabfüller geliefert wird. Dieser zieht lediglich die wachsdurchtränkte Papierfolie ab und plaziert das Etikett 2 auf dem Behälter 1.

Es ist auch möglich, den Etikettstreifen 6 ebenfalls mit einer Klebeschicht zu versehen, wobei die Klebeschicht des Etikettstreifens 6 eine separate, abdeckende Papierfolie aufweist, die beim Aufbringen des Etikettes 2 auf dem Behälter 1 auf dem Etikettstreifen 6 verbleibt, damit der Etikettstreifen 6 nicht zusammen mit dem Etikett 2 auf dem Behälter 1 befestigt wird.

Eine andere Möglichkeit besteht darin, den Etikettstreifen 6 auf seiner Innenseite mit einer Verstärkungsschicht zu versehen, die vorzugsweise aus einem textilen Gewebe besteht und mit dem Etikettstreifen 6 verklebt ist. Dadurch wird der Etikettstreifen 6 zusätzlich verstärkt.

Zur Vermeidung von Einrissen im Bereich der Schlitzenden sind diese jeweils mit einer Ausnehmung 7 versehen, die bereits herstellerseitig ausgestanzt wird.

Da der Etikettstreifen 6 nicht mit der Außenfläche des Behälters 1 verklebt ist, kann der Etikettstreifen 6 in Pfeilrichtung weggeklappt werden. Dabei erfolgt eine Knickung im Bereich der Ausnehmung 7 bzw. im in Schlitzrichtung verlängerten Bereich des Etikettes 2. Der Etikettstreifen 6 überspannt dabei den Behälterboden 3 und bildet zwischen der Unterseite des Etikettstreifens 6 und dem Behälterboden 3 einen Abstand, in den eine Aufhängevorrichtung 8, beispielsweise eines Infusionsständers, eingreifen kann. Durch Einhängen des Etikettstreifens 6 in die Aufhängevorrichtung 8 wird die Über-Kopf-Aufhängung des Behälters 1 gewährleistet, so daß ein auf dem Behälter befindlicher Deckel 9 an einer entsprechend dafür vorgesehenen Stelle, beispielsweise durch eine Injektionsnadel oder eine Infusionskanüle durchstoßen werden kann, so daß diese mit dem Innenraum des Behälters 1 in Verbindung gebracht werden können. Die Tragfähigkeit des Etikettstreifens wird durch das Volumen des Behälters 1 gemäß DIN 58369 mit 30 bzw. 50 N bestimmt.

**Ansprüche**

1. Über Kopf aufzuhängender Behälter aus Glas oder Kunststoff, insbesondere Flasche zum Verabreichen von medizinischen Flüssigkeiten an einen Patienten, mit einem Etikett, auf dessen Rückseite eine stark haftende Klebeschicht aufgebracht ist, dadurch gekennzeichnet, daß das Etikett (2) mit seiner dem Behälterboden (3) zugewandten Seite bis an die Bodenebene (4) heranreicht und mit seiner Breite mindestens die Hälfte des Behälterumfanges umgibt, und daß parallel zur Bodenebene (4) nahezu über seine gesamte Breite geschlitzt ist, so daß ein Etikettstreifen (6) gebildet wird, wobei sich der vom Schlitz (5) senkrecht parallel zur Bodenebene (4) erstreckende Etikettstreifen (6) über den Behälterboden (3) wegklappbar ausgebildet und an einer Aufhängevorrichtung (8) aufhängbar ist.

2. Behälter nach Anspruch 1, dadurch gekennzeichnet, daß das Etikett (2) mit seiner Breite bis zu zwei Drittel des Behälterumfanges umgibt.

3. Behälter nach Anspruch 1, dadurch gekennzeichnet, daß der Etikettstreifen (6) ohne Klebeschicht ausgebildet ist.

4. Behälter nach Anspruch 1, dadurch gekennzeichnet, daß der vom Schlitz (5) zur Bodenebene (4) gerichtete Etikettstreifen (6) eine Verstärkungsschicht aufweist.

5. Behälter nach Anspruch 4, dadurch gekennzeichnet, daß die Verstärkungsschicht eine textile Schicht ist.

6. Behälter nach Anspruch 4 und 5, dadurch gekennzeichnet, daß der vom Schlitz (5) zur Bodenebene (4) gerichtete Etikettstreifen (6) mit der Verstärkungsschicht vorzugsweise verklebt ist.

7. Behälter nach Anspruch 1, dadurch gekennzeichnet, daß am jeweiligen Schlitzende zur Vermeidung von Einrissen eine Ausnehmung (7) vorgesehen ist.

8. Behälter nach Anspruch 1, dadurch gekennzeichnet, daß das Etikett (2) aus Polyethylen, Polypropylen, Polyurethan oder aus Laminaten mit Polyester oder Polyamid besteht.

9. Behälter nach Anspruch 1, dadurch gekennzeichnet, daß das Etikett (2) aus kunststoffkaschiertem Papier besteht.

10. Behälter nach Anspruch 1 bis 9, dadurch gekennzeichnet, daß die Tragfähigkeit des Etikettstreifens (6) durch das Behältervolumen mit 30 bzw. 50 N bestimmt wird.

11. Behälter nach Anspruch 1 bis 10, dadurch gekennzeichnet, daß das Etikett (2) eine Reißfestigkeit von etwa 40 bis etwa 110 N/mm² in Abhängigkeit von der Foliendicke und vom Folienmaterial aufweist.

12. Behälter nach Anspruch 1 bis 10, dadurch gekennzeichnet, daß das Etikett (2) eine Reißkraft von etwa 30 bis etwa 120 N/15 mm in Abhängigkeit von der Foliendicke und vom Folienmaterial aufweist.